# EUROPEAN PATENT SPECIFICATION

(11) **EP 2 239 319 B1**
(45) Date of publication and mention of the grant of the patent: **06.11.2013**
(21) Application number: 09703248.6
(22) Date of filing: 23.01.2009
(51) Int. Cl.: C12N 1/20, A01G 1/00, A01G 7/00

(54) **METHOD AND BACTERIUM FOR PROMOTING THE GROWTH OF RACOMITRIUM CANESCENS AND SEED PLANTS**
VERFAHREN UND BAKTERIUM ZUR FÖRDERUNG DES WACHSTUMS VON RACOMITRIUM CANESCENS UND SAMENPFLANZEN
PROCÉDÉ ET BACTÉRIE FAVORISANT LA CROISSANCE DE RACOMITRIUM CANESCENS ET DE SPERMATOPHYTE

(30) Priority: 23.01.2008 JP 2008012159; 08.05.2008 JP 2008122462
(43) Date of publication of application: 13.10.2010
(73) Proprietor: National University Corporation Okayama University, Kita-ku Okayama-shi Okayama 700-8530 (JP); Kinki University, Higashiosaka-shi Osaka 577-8502 (JP)
(72) Inventor: TANI, Akio, Kurashiki-shi Okayama 710-0046 (JP); AKITA, Motomu, Kinokawa-shi Wakayama 649-6493 (JP)
(74) Representative: Isarpatent
(86) International application number: PCT/JP2009/051034
(87) International publication number: WO 2009/093675

(56) References cited:
- WO-A2-01/90303
- JP-A- 2005 040 114
- US-A- 5 961 687
- HORNSCHUH M ET AL: "Moss-associated methylobacteria as phytosymbionts: an experimental study", NATURWISSENSCHAFTEN, SPRINGER, BERLIN, DE, vol. 93, no. 10, 12 July 2006 (2006-07-12) , pages 480-486, XP019413157, ISSN: 1432-1904, DOI: DOI:10.1007/S00114-006-0137-7
- HORNSCHUH M ET AL: "Epiphytic bacteria associated with the bryophyte Funaria hygrometrica: Effects of Methylobacterium strains on protonema development.", PLANT BIOLOGY (STUTTGART), vol. 4, no. 6, November 2002 (2002-11), pages 682-687, XP002615039, ISSN: 1435-8603
- ABANDA-NKPWATT, D ET AL.: 'Molecular interaction between Methylobacterium extorquens and seedlings: growth promotion, methanol consumption, and localization of the methanol emission site' JOURNAL OF EXPERIMENTAL BOTANY vol. 57, no. 15, December 2006, pages 4025 - 4032, XP008129500
- 'Dai 60 Kai Abstracts of the Annual Meeting of the Society for Biotechnology, Japan, 11 July, 2008', 11 July 2008 article AKIO TANI ET AL.: 'Racomitrium Canescens no Seiiku o Sokushin suru Biseibutsu', page 211

### SEQUENCE LISTING

<110> NATIONAL UNIVERSITY CORPORATION OKAYAMA UNIVERSITY
   KINKI UNIVERSITY
   TANI, Akio
   AKITA, Motomu
<120> Method for promotion of growth of Racomitrium canescens and seed plants
   and bacteria for growth promotion
<130> GP118-PCT
<160> 9
<170> PatentIn version 3.1
<210> 1
   <211> 60
   <212> DNA
   <213> Artificial
<220>
   <223> Forward primer
<400> 1
   cgcccgccgc gcgcggcggg cggggcgggg gcacgggggg actcctacgg gaggcagcag 60
<210> 2
   <211> 16
   <212> DNA
   <213> Artificial
<220>
   <223> Reverse primer
<400> 2
   attaccgcgg ctgctg 16
<210> 3
   <211> 1433
   <212> DNA
   <213> Methylobacterium extorquens
<400> 3
   ggctagagcg acgctggctg taggcttatc acatgcttgt cgaacgggca ccttcgggtg     60
   tcagtggcag acgggtgagt aacacgtggg aacgtaccct tcggttcgga ataactcagg    120
   gaaacttgag ctaataccgg atacgccctt ttggggaaag gtttactgcc gaaggatcgg    180
   cccgcgtctg attagcttgt tggtggggta acggcctacc aaggcgacga tcagtagctg    240
   gtctgagagg atgatcagcc acactgggac tgagacacgg cccagactcc tacgggaggc    300
   agcagtgggg aatattggac aatgggcgca agcctgatcc agccatgccg cgtgagtgat    360
   gaaggcctta gggttgtaaa gctcttttgt ccgggacgat aatgacggta ccggaagaat    420
   aagccccggc taacttcgtg ccagcagccg cggtaatacg aagggggcta gcgttgctcg    480
   gaatcactgg gcgtaaaagg gcgcgtaggc ggccgattaa gtcgggggtg aaagcctgtg 540
   gctcaaccac agaattgcct tcgatactgg ttggcttgag atcggaagag gacagcggaa 600
   ctgcgagtgt agaggtgaaa ttcgtagata ttcgcaagaa caccagtggc gaaggcggct 660
   gtctggtccg gttctgacgc tgaggcgcga aagcgtgggg agcaaacagg attagatacc 720
   ctggtagtcc acgccgtaaa cgatgaatgc cagccgttgg cctgcttgca ggtcagtggc 780
   gccgctaacg cattaagcat tccgcctggg gagtacggtc gcaagattaa aactcaaagg 840
   aattgacggg ggcccgcaca agcggtggag catgtggttt aattcgaagc aacgcgcaga 900
   accttaccat cccttgacat ggcatgttac ctcgagagat cggggatcct cttcggaggc 960
   gtgcacacag gtgctgcatg ggctgtcgtc agctcgtgtc gtgagatgtt gggttaagtc 1020
   cccaacgagc gcaatcccac gtccttagtt gccatcattc agttgggcac tctatggaga 1080
   ctgccggtga taagccgcga ggaaggtgtg gatgacgtca agtcctcatg gcccttacgg 1140
   gatgggctac acacgtgcta caatggcggt gacagtggga cgcgaagccg cgaggtggag 1200
   caaatcccca aaaaccgtct cagttcggat tgcactctgc aactcgggtg catgaaggcg 1260
   gaatcgctag taatcgtgga tcagcacgcc acggtgaata cgttcccggg ccttgtacac 1320
   accgcccgtc acaccatggg agttggtctt acccgacggc gctgcgccaa ccgcaaggag 1380
   gcaggcgacc acggtagggt cagcgactgg ggtgaagtcg taacaaggta gcc 1433
<210> 4
   <211> 1446
   <212> DNA
   <213> Methylobacterium extorquens
<400> 4
   agagtttgat cctggctcag agcgaacgct ggcggcaggc ttaacacatg caagtcgaac 60
   gggcaccttc gggtgtcagt ggcagacggg tgagtaacac gtgggaacgt acccttcggt 120
   tcggaataac tcagggaaac ttgagctaat accggatacg cccttttggg gaaaggttta 180
   ctgccgaagg atcggcccgc gtctgattag cttgttggtg gggtaacggc ctaccaaggc 240
   gacgatcagt agctggtctg agaggatgat cagccacact gggactgaga cacggcccag 300
   actcctacgg gaggcagcag tggggaatat tggacaatgg gcgcaagcct gatccagcca 360
   tgccgcgtga gtgatgaagg ccttagggtt gtaaagctct tttgtccggg acgataatga 420
   cggtaccgga agaataagcc ccggctaact tcgtgccagc agccgcggta atacgaaggg 480
   ggctagcgtt gctcggaatc actgggcgta aagggcgcgt aggcggccga ttaagtcggg 540
   ggtgaaagcc tgtggctcaa ccacagaatt gccttcgata ctggttggct tgagaccgga 600
   agaggacagc ggaactgcga gtgtagaggt gaaattcgta gatattcgca agaacaccag 660
   tggcgaaggc ggctgtctgg tccggttctg acgctgaggc gcgaaagcgt ggggagcaaa 720
   caggattaga taccctggta gcccacgccg taaacgatga atgccagccg ttggcctgct 780
   tgcaggtcag tggcgccgct aacgcattaa gcattccgcc tggggagtac ggtcgcaaga 840
   ttaaaactca aaggaattga cgggggcccg cacaagcggt ggagcatgtg gtttaattcg 900
   aagcaacgcg cagaacctta ccatcccttg acatggcatg ttacctcgag agatcgggga 960
   tcctcttcgg aggcgtgcac acaggtgctg catggctgtc gtcagctcgt gtcgtgagat 1020
   gttgggttaa gtcccgcaac gagcgcaacc cacgtcctta gttgccatca ttcagttggg 1080
   cactctaggg agactgccgg tgataagccg cgaggaaggt gtggatgacg tcaagtcctc 1140
   atggccctta cgggatgggc tacacacgtg ctacaatggc ggtgacagtg ggacgcgaag 1200
   ccgcgaggtg gagcaaatcc ccaaaaaccg tctcagttcg gattgcactc tgcaactcgg 1260
   gtgcatgaag gcggaatcgc tagtaatcgt ggatcagcac gccacggtga atacgttccc 1320
   gggccttgta cacaccgccc gtcacaccat gggagttggt cttacccgac ggcgctgcgc 1380
   caaccgcaag gaggcaggcg accacggtag ggtcagcgac tggggtgaag tcgtaacaag 1440
   gtagcc 1446
<210> 5
   <211> 544
   <212> DNA
   <213> Spirosoma sp.
<400> 5
   agagtttgat cctggctcag gatgaacgct agcggcaggc ctaatacatg caattcgaac 60
   gggtcgcaag accagtggca aacgggtcgc gtaacgcgta agcaacctgc ctcatactgg 120
   gggatagccc ggcgaaagct ggggtaaacc cgcacggtcc gattgagtca cctggctcga 180
   tgggtaaaca tttatgggta tgagaggggc ttgcgtctga ttagttagtt ggcagggtaa 240
   cggcctacca agacgatgat cagtaggggt tctgagggga ttggccccca catgggtact 300
   gagatacgga cccaactcct acgggaggca gcagtaggga atattgggca atggaggcaa 360
   ctctgaccca gccatgccgc gtgcaggatg aaggcgctca gcgttgtaaa ctgcttttat 420
   tggtgaagaa ctgtagtcct gcgggattac ttgacggtag ccaaggaata agcaccggct 480
   aactccgtgc cagcagccgc ggtaatacgg agggtgcaag cgttgtccgg atttattggg 540
   ttta 544
<210> 6
   <211> 1424
   <212> DNA
   <213> Methylobacterium extorquens
<400> 6
   cgcctgtcgg caggctctaa cacatgcaag tcgaactggg taaccttcgg gtgtcagtgg     60
   cagactggtg agtaacacgt gggaacgtac ccttcggttc ggaataactc agggaaactt    120
   gagctaatac cggatacgcc cttttgggga aaggtttact gccgaaggat cggcccgcgt    180
   ctgattagct tgttggtggg gtaacggcct accaaggcga cgatcagtag ctggtctgag    240
   aggatgatca gccacactgg gactgagaca cggcccagac tcctacggga ggcagcagtg    300
   gggaatattg gacaatgggc gcaagcctga tccagccatg ccgcgtgagt gatgaaggcc    360
   ttagggttgt aaagctcttt tgtccgggac gataatgacg gtaccggaag aataagcccc    420
   ggctaacttc gtgccagcag ccgcggtaat acgaaggggg ctagcgttgc tcggaatcac    480
   tgggcgtaaa gggcgcgtag gcggccgatt aagtcggggg tgaaagcctg tggctcaacc    540
   acagaattgc cttcgatact ggttggcttg agatcggaag aggacagcgg aactgcgagt    600
   gtagaggtga aattcgtaga tattcgcaag aacaccagtg gcgaaggcgg ctgtctggtc    660
   cggttctgac gctgaggcgc gaaagcgtgg ggagcaaaca ggattagata ccctggtagt    720
   ccacgccgta aacgatgaat gccagccgtt ggcctgcttg caggtcagtg gcgccgctaa    780
   cgcattaagc attccgcctg gggagtacgg tcgcaagatt aaaactcaaa ggaattgacg    840
   ggggcccgca caagcggtgg agcatgtggt ttaattcgaa gcaacgcgca gaaccttacc    900
   atcccttgac atggcatgtt acctcgagag atcggggatc ctcttcggag gcgtgcacac    960
   aggtgctgca tggctgtcgt cagctcgtgt cgtgagatgt tgggttaagt cccgcaacga   1020
   gcgcaaccca cgtccttagt tgccatcatt cagttgggca ctctagggag actgccggtg   1080
   ataagccgcg aggaaggtgt ggatgacgtc aagtcctcat ggcccttacg ggatgggcta   1140
   cacacgtgct acaatggcgg tgacagtggg acgcgaagcc gcgaggtgga gcaaatcccc   1200
   aaaaaccgtc tcagttcgga ttgcactctg caactcgggt gcatgaaggc ggaatcgcta   1260
   gtaatcgtgg atcagcacgc cacggtgaat acgttcccgg gccttgtaca caccgcccgt   1320
   cacaccatgg gagttggcct tacccgacgg cgctgcgcca accgcaagga ggcaggcgac   1380
   cacggtaggg tcagcgactg gggtgaagtc gtaacaaggt aacc                     1424
<210> 7
   <211> 1446
   <212> DNA
   <213> Methylobacterium extorquens
<400> 7
   agagtttgat cctggctcag agcgaacgct ggcggcaggc ttaacacatg caagtcgaac     60
   gggcaccttc gggtgtcagt ggcagacggg tgagtaacac gtgggaacgt acccttcggt    120
   tcggaataac tcagggaaac ttgagctaat accggatacg cccttttggg gaaaggttta    180
   ctgccgaagg atcggcccgc gtctgattag cttgttggtg gggtaacggc ctaccaaggc    240
   gacgatcagt agctggtctg agaggatgat cagccacact gggactgaga cacggcccag    300
   actcctacgg gaggcagcag tggggaatat tggacaatgg gcgcaagcct gatccagcca    360
   tgccgcgtga gtgatgaagg ccttagggtt gtaaagctct tttgtccggg acgataatga    420
   cggtaccgga agaataagcc ccggctaact tcgtgccagc agccgcggta atacgaaggg    480
   ggctagcgtt gctcggaatc actgggcgta aagggcgcgt aggcggccga ttaagtcggg    540
   ggtgaaagcc tgtggctcaa ccacagaatt gccttcgata ctggttggct tgagaccgga    600
   agaggacagc ggaactgcga gtgtagaggt gaaattcgta gatattcgca agaacaccag    660
   tggcgaaggc ggctgtctgg tccggttctg acgctgaggc gcgaaagcgt ggggagcaaa    720
   caggattaga taccctggta gtccacgccg taaacgatga atgccagccg ttggcctgct    780
   tgcaggtcag tggcgccgct aacgcattaa gcattccgcc tggggagtac ggtcgcaaga    840
   ttaaaactca aaggaattga cgggggcccg cacaagcggt ggagcatgtg gtttaattcg    900
   aagcaacgcg cagaacctta ccatcccttg acatggcatg ttacctcgag agatcgggga    960
   tcctcttcgg aggcgtgcac acaggtgctg catggctgtc gtcagctcgt gtcgtgagat   1020
   gttgggttaa gtcccgcaac gagcgcaacc cacgtcctta gttgccatca ttcagttggg   1080
   cactctaggg agactgccgg tgataagccg cgaggaaggt gtggatgacg tcaagtcctc   1140
   atggccctta cgggatgggc tacacacgtg ctacaatggc ggtgacagtg ggacgcgaag   1200
   ccgcgaggtg gagcaaatcc ccaaaaaccg tctcagttcg gattgcactc tgcaactcgg   1260
   gcgcatgaag gcggaatcgc tagtaatcgt ggatcagcac gccacggtga atacgttccc   1320
   gggccttgta cacaccgccc gtcacaccat gggagttggt cttacccgac ggcgctgcgc   1380
   caaccgcaag gaggcaggcg accacggtag ggtcagcgac tggggtgaag tcgtaacaag   1440
   gtagcc                                                               1446
<210> 8
   <211> 1445
   <212> DNA
   <213> Methylobacterium aquaticum
<400> 8
   agagtttgat cctggctcag agcgaacgct ggcggcaggc ttaacacatg caagtcgagc     60
   gggcccttcg gggtcagcgg cagacgggtg agtaacgcgt gggaacgtgc ccttcggttc    120
   ggaataactc agggaaactt gagctaatac cggatacgcc cttatgggga aaggtttatc    180
   tgccgaagga tcggcccgcg tctgattagc tagttggtga ggttacggct caccaaggcg    240
   acgatcagta gctggtctga gaggatgatc agccacactg ggactgagac acggcccaga    300
   ctcctacggg aggcagcagt ggggaatatt ggacaatggg ggcaaccctg atccagccat    360
   gccgcgtgag tgatgacggc cttagggttg taaagctctt ttctccggga cgataatgac    420
   ggtaccggag gaataagccc cggctaactt cgtgccagca gccgcggtaa tacgaagggg    480
   gctagcgttg ctcggaatca ctgggcgtaa agggcgcgta ggcggctgat ttaatcgagg    540
   gtgaaagccc gtggctcaac cacggaatgg ccttcgatac tgattggctt gagaccggaa    600
   gaggacagcg gaactgcgag tgtagaggtg aaattcgtag atattcgcaa gaacaccagt    660
   ggcgaaggcg gctgtctggt ccggttctga cgctgaggcg cgaaagcgtg gggagcaaac    720
   aggattagat accctggtag tccacgctgt aaacgatgaa tgctagccgt tggggtgcat    780
   gcacctcagt ggcgccgcta acgctttaag cattccgcct ggggagtacg gtcgcaagat    840
   taaaactcaa aggaattgac gggggcccgc acaagcggtg gagcatgtgg tttaattcga    900
   agcaacgcgc agaaccttac catcccttga catggcatgc gagccggaga gatccggtgt    960
   tcccttcggg gacgtgcaca caggtgctgc atggctgtcg tcagctcgtg tcgtgagatg   1020
   ttgggttaag tcccgcaacg agcgcaaccc acgtcctcag ttgccatcat ttggttgggc   1080
   actctgggga gactgccggt gataagccgc gaggaaggtg tggatgacgt caagtcctca   1140
   tggcccttac ggggtgggct acacacgtgc tacaatggcg gtgacaatgg gcagcgaagg   1200
   ggcgacctgg agcgaatccc caaaagccgt ctcagttcgg attgcactct gcaactcggg   1260
   tgcatgaagg cggaatcgct agtaatcgtg gatcagcatg ccacggtgaa tacgttcccg   1320
   ggccttgtac acaccgcccg tcacaccatg ggagttggtc ttacccgacg gcgctgcgcc   1380
   aaccgcaagg aggcaggcga ccacggtagg gtcagcgact ggggtgaagt cgtaacaagg   1440
   tagcc                                                               1445
<210> 9
   <211> 621
   <212> DNA
   <213> Mesorhizobium sp.
<400> 9
   agagtttgat cctggctcag aacgaacgct ggcggcaggc ttaacacatg caagtcgagc     60
   gcctcgcaag aggagcggca gacgggtgag taacgcgtgg gaatctaccc atctctacgg     120
   aacaactccg ggaaactgga gctaataccg tatacgtcct tcgggagaaa gatttatcgg    180
   agatggatga gcccgcgttg gattagctag ttggtggggt aatggcctac caaggcgacg    240
   atccatagct ggtctgagag gatgatcagc cacattggga ctgagacacg gcccaaactc    300
   ctacgggagg cagcagtggg gaatattgga caatgggcga aagcctgatc cagccatgcc    360
   gcgtgagtga tgaaggccct agggttgtaa agctctttca acggtgaaga taatgacggt    420
   aaccgtagaa gaagccccgg ctaacttcgt gccagcagcc gcggtaatac gaagggggct    480
   agcgttgttc ggaattactg ggcgtaaagc gcacgtaggc ggattgttaa gttaggggtg    540
   aaatcccagg gctcaaccct ggaactgcct ttaatactgg caatctcgag tccgagagag    600
   gtgagtggaa ttccgagtgt a                                              621

## Description

### TECHNICAL FIELD

The present invention relates to growth promotion of Racomitrium canescens and more specifically, to bacteria having a property to promote growth of Racomitrium canescens plants, a composition comprising Racomitrium canescens and some of the bacteria, and a method for promoting growth of Racomitrium canescens by letting Racomitrium canescens and the bacteria live symbiotically.

### BACKGROUND ART

Though the bryophytes [Bryophyta] fix CO₂ and release oxygen through the process of carbon dioxide assimilation in the same manner as other plants (patent document 1), they, in the long run, have a higher CO₂ fixation ability than deciduous plants because while deciduous plants' fallen leaves are easily decomposed by microorganisms to release CO₂, the bryophytes, even after they die, are very slow to decay and therefore also very slow to release CO₂. So far, cultivation of bryophytes has been done under a condition where they are nearly left in the nature. However, this approach has a problem that their growth rate greatly depends on weather, and, further, a difficulty that their growth is so slow that it requires more than one year for planted bryophytes to form a community in the place where they were planted. Among bryophytes, Racomitrium canescens draws particular attention as greening material from such viewpoints that it is strong against dryness, grows even in a sunny place, requires no soil as a base, does not need maintenance, and the like (patent documents 2, 3, 4 and 5). However, it is the bottleneck against its wide use for greening that the growth of bryophytes is very slow. Consequently, there is a potential need for technology to promote growth of Racomitrium canescens.

Turning to other plants than the bryophytes, promoting growth of agriculture plants, for example, i.e., plants produced as food or animal feed, also leads to shorter time period until harvesting as well as to increased yield. Thus, such growth-promoting technology would be useful, and there is a potential need for the technology. It is known that Methylobacterium extorquens has a growth-promoting effect on Nicotiana tabacum, which is a species of tobacco (non-patent document 1), and that M. extorquens has a growth-promoting effect on barley (patent documents 6), and that M. extorquens also has a growth-promoting effect on soybean (patent documents 6-11). However, the promoting effects are not sufficient, and thus it is desirable that other bacteria having promoting effect become available.

- [patent document 1]: Japanese Patent Application Publication No. 2006-254900
- [patent document 2]: Japanese Patent Application Publication No. 2006-006292
- [patent document 3]: Japanese Patent Application Publication No. 2004-236518
- [patent document 4]: Japanese Patent Application Publication No. 2002-360060
- [patent document 5]: Japanese Patent Application Publication No. H07-227142
- [patent document 6]: U.S. Patent Application Publication No. 20030211082
- [patent document 7]: U.S. Patent. No. 5512069
- [patent document 8]: U.S. Patent. No. 5961687
- [patent document 9]: U.S. Patent. No. 6174837
- [patent document 10]: U.S. Patent Application Publication No. 20010001095
- [patent document 11]: U.S. Patent Application Publication No. 20060228797
- [non-patent document 1]: Journal of Experimental Botany, 57(15), p.4025-4032 (2006)
D1, Hornschuh M. et al. "Moss-associated methylobacteria as phytosymbionts; an experimental study", Naturwissenschaften, Springer, Berlin, DE, vol. 93, no. 10,12 July 2006, pages 480-486, XP019413157, ISSN 1432-1904; KOI: KOI: 10.1007/S00114-006-0137-7 describes three methanol-utilizing bacteria of the genus Methylobacterium and the effect of these bacteria on the growth of protonemata of the moss *Funaria hygrometrica.* It could be shown that normal protonema development in *Funaria hygrometrica* is promoted by the naturally occurring Methylobacterium associated with the plant cells, and the conclusion was drawn that the phytohormone-producing methylobacteria associated with *Funaria hygrometrica* and related moss species has to be regarded as phytosymbionts.

### DISCLOSURE OF THE INVENTION

### PROBLEM TO BE SOLVED BY THE INVENTION

Against the above background, the present invention is intended to provide a means to promote growth of Racomitrium canescens.

### MEANS TO SOLVE THE PROBLEM

The present inventors assumed the presence of a symbiotic relation between Racomitrium canescens and other microorganisms, and have conducted a study seeking for such microorganisms. After having attempted to isolate bacteria from Racomitrium canescens collected, they have found the presence of methanol-utilizing bacteria. Symbiosis of Racomitrium canescens and methanol-utilizing bacteria has not been known so far. The inventors, assuming that the methanol-utilizing bacteria occur as dominant species for Racomitrium canescens, investigated what function they had on Racomitrium canescens. As a result, the inventors have found that there are some strains among the bacteria belonging to Methylobacterium aquaticum or Methylobacterium extorquens which act to promote growth of protonemata of Racomitrium canescens, and have isolated and identified those strains. It has not been known before that these two kinds of bacteria include strains which promote growth of protonemata of Racomitrium canescens. Based on these findings, the inventors have conducted a further study and completed the present invention. Namely, the invention provides the following.

1. A bacterium selected from methanol-utilizing bacteria of the genus Methylobacterium which bacteria are deposited under designated accession numbers FERM BP-11078, FERM BP-11079, FERM BP-11080 and FERM BP-11071, respectively.
2. A composition comprising bacterium of 1 above and protonemata of Racomitrium canescens.
3. A composition according to 2 above, wherein the bacterium and the protonemata are contained in a culture medium.
4. A method for growth promotion of protonemata of Racomitrium canescens, which method comprises a step of providing at least one of methanol-utilizing bacteria of the genus Methylobacterium which bacteria are deposited under designated accession numbers FERM BP-11078, FERM BP-11079, FERM BP-11080, and FERM BP-11071, respectively, and a step of culturing protonemata of Racomitrium canescens together with the provided bacterium or bacteria.

### EFFECT OF THE INVENTION

According to the present invention, growth of protonemata of Racomitrium canescens can be promoted. Consequently, according to the invention, it enables to overcome the difficulty of Racomitrium canescens that it is slow to grow, and thus to grow the protonemata quickly. Consequently, by using the invention for agriculture, it is possible to shorten a time period until harvest or to increase the yield.

### BRIEF DESCRIPTION OF DRAWINGS

Figure 1 is a photograph substituted for a drawing, showing the results of DGGE.
Figure 2 is photographs substituted for a drawing, showing growth comparison results of protonemata by types of and with or without inoculation of bacteria.
Figure 3 is a graph showing growth comparison results of protonemata by types of and with or without inoculation of bacteria.
Figure 4 is a photograph substituted for a drawing, showing growth inhibition of molds by MA-22A.

### BEST MODE FOR CARRYING OUT THE INVENTION

Among the methanol-utilizing bacteria of the genus Methylobacterium of the present invention, those named MA-22A, MC-21B and MC-21C were deposited domestically on November 28, 2007 to International Patent Organism Depositary (IPOD), National Institute of Advanced Industrial Science and Technology, Independent Administrative Agency, at Tsukuba Center Chuo No.6, 1-1-1, Higashi, Tsukuba-shi, Ibaraki, Japan, and accession numbers of FERM P-21449, FERM P-21450 and FERM P-21451 were designated thereto, respectively, and then, those deposits were changed to international deposits based on the Budapest Treaty on December 16, 2008 at IPOD, and accession numbers of FERM BP-11078, FERM BP-11079 and FERM BP-11080 were designated thereto, respectively. Also, bacterium named MC-11A was deposited internationally to IPOD on December 10, 2008, and accession number of FERM BP-11071 was designated thereto.

Properties of MA-22A, MC-21B, MC-21C and MC-11A are shown in the following Table 1a and Table 1b.

[Table 1a]

**Table 1a. Deposited Bacterial Strains**

| Name of Strain | MA-22A | MC-21B | MC-21C |
|---|---|---|---|
| Name of Bacterium | *Methylobacterium aquaticum* | *Methylobacterium extorquens* | *Methylobacterium extorquens* |
| Designated Accession Number | FERMBP-11078 | FERM BP-11079 | FERM BP-11080 |
| Oxygen Requirement | aerobic | aerobic | aerobic |
| Light Requirement | unnecessary | unnecessary | unnecessary |
| | Color: pink | Color: pink | Color: pink |
| | Form: circular | Form: circular | Form: circular |
| | Bulging State: hemispheric | Bulging State: hemispheric | Bulging State: hemispheric |
| | Circumference: entire | Circumference: entire | Circumference: entire |
| | Surface: smooth | Surface: smooth | Surface: smooth |
| | Transparency: opaque | Transparency: opaque | Transparency: opaque |
| Colony Morphology | Viscosity: butter like | Viscosity: butter like | Viscosity: butter like |
| | Change of Colony Morphology by Mutation: non Change of Colony Morphology by Culturing Condition/ Physiological | Change of Colony Morphology by Mutation: non Change of Colony Morphology by Culturing Condition/Physiological | Change of Colony Morphology by Mutation: non Change of Colony Morphology by Culturing Condition/Physiological |
| | Condition: non | Condition: non | Condition: non |
| Methanol-utilizing Ability | + | + | + |

[Table 1b]

**Table 1b. Deposited Bacterial Strains**

| Name of Strain | MC-11A |
|---|---|
| Name of Bacterium | *Methylobacterium extorquens* |
| Designated Accession Number | FERM BP-11071 |
| Oxygen Requirement | aerobic |
| Light Requirement | unnecessary |
| | Color: pink |
| | Form: circular |
| | Bulging State: hemispheric |
| | Circumference: entire |
| | Surface: smooth |
| | Transparency: opaque |
| Colony Morphology | Viscosity: butter like |
| | Change of Colony Morphology by Mutation: non Change of Colony Morphology by Culturing Condition/Physiological |
| | Condition: non |
| Methanol-utilizing Ability | + |

Each bacterium above is that which was isolated from Racomitrium canescens in nature. In order to use these bacteria for growth promotion of protonemata of Racomitrium canescens, it is enough, for example, to prepare in a medium at least one of these bacteria before culturing protonemata of Racomitrium canescens, and then inoculate the protonemata with the bacterium or bacteria. In this case, the culture may be performed, for example, using the Culture Medium Y (including 0.8% agar) described in the part of the Examples in a light place (e.g., under a fluorescent lamp) at high humidity (e.g., 100%) and at 20 °C, though the condition is not limited thereto, and culture may be performed under an appropriate condition. The amount of bacterium or bacteria with which the protonemata of Racomitrium canescens were inoculated may be determined as desired with no particular limitation. For example, growth promotion is observed by inoculating 1/30 of bacteria quantity in a colony (approximately 1 mm in diameter) per protonema. Since these bacteria easily proliferate when they are in the vicinity of protonema of Racomitrium canescens, the quantity to be inoculated may be less than this as far as subsequent growth of the bacteria is observed. Each of the above bacteria is pink-colored, and their growth can be clearly observed with the naked eye as a pink region surrounding a protonema. As far as growth of inoculated bacteria can be observed through culture, only the first inoculation is enough, though additional inoculation may be made as desired. A large quantity of protonemata to be let differentiate into gametophytes can be obtained in a short period of time by making protonemata quickly grow in this way.

### EXAMPLES

This invention is described below in detail based on examples, though it is not intended that the invention be limited to the examples.

### [Example 1] Growth promotion of Racomitrium canescens (1)

### [Sterilization of Racomitrium canescens]

Naturally grown moss of Racomitrium canescens (grown in Yamagata Prefecture) was obtained. The sporangia were washed with water of sufficient quantity (more than 1 mL per sporangium) twice, and treated with 70% ethanol for one minute. After having been further immersed in an aqueous solution containing 1% sodium perchlorate and 0.5% Tween20 for 20 minutes, the sporangia were washed with sterilized water five times. The sporangia were broken in sterilized water, and spores flowing out were collected with pipette. Sterile spores thus obtained were spread to an agar culture medium (a liquid of Hyponex ™* diluted 1000 folds with water + 0.8% agar), and cultured under a fluorescent lamp at 20 °C. Growth of protonemata was observed with the naked eye in approximately two weeks after the start of culture. Note: Hyponex [mfd. by HYPONEX JAPAN CORP., LTD, containing 6% nitrogen in total (of which, 2.90% ammonium-nitrogen, 1.05% nitrate-nitrogen), 10.0% water soluble phosphate, 5.0% water soluble potassium, 0.05% water-soluble magnesia, 0.001% water-soluble manganese , 0.005% water-soluble boron]

The culture was performed also in the following Culture Medium Y (containing 0.8% agar), and growth of protonemata was equally observed with the naked eye. These sterile protonemata were used in experiments of growth promotion by microorganisms.

[Table 2]

**Table 2. Composition of Culture Medium Y**

| Component | Quantity Contained (mM) |
|---|---|
| NH₄NO₃ | 1.25 |
| KCl | 2.68 |
| CaCl₂/2H₂O | 1.77 |
| MgSO₄/7H₂O | 0.81 |
| KH₂PO₄ | 1.47 |
| Na₂EDTA | 0.181 |
| FeSO₄/7H₂O | 0.09 |
| H₃BO₃ | 0.024 |
| MnSO₄/7H₂O | 0.099 |

### [Search for microorganisms from Racomitrium canescens]

The above naturally grown moss of Racomitrium canescens (before sterilization) was suspended in sterilized water, added to 1L of liquid culture medium (Hyponex ™ diluted 1000 folds with water), and then stirred at approximately 18 °C for seven weeks under a fluorescent lamp (30 cm distance, 16 hours/day). Each sample of culture medium was collected before stirring, after four and seven weeks of stirring. Each sample obtained was cultured using plates made of 1/3 LB agar medium (3.3 g/L polypeptone, 1.6 g/L yeast extract, 10 g/L sodium chloride, 15 g/L agar) and plates of 1000 fold-diluted Hyponex which was solidified with agar (1.5%). A number of various colonies were isolated, and each of them was subjected to identification of bacteria and to examination of their property. However, none of the bacteria was found to promote growth of Racomitrium canescens.

Then, the above naturally grown moss of Racomitrium canescens (before sterilization) was suspended in sterilized water, and cultured under each of the above culture conditions. The culture was sampled at random four and seven weeks after the start of the culture, and then subjected to DGGE (denaturing gradient gel electrophoresis) analysis. That is, 5mL of each culture were centrifuged (10,000×g, ten minutes) into precipitate and supernatant. The precipitate was collected and washed with a 0.85% sodium chloride solution, and, then, suspended in one mL of BL buffer [40mM Tris-HCl, 1% Tween20, 0.5% Nonidet P-40 (mfd. by Nacalai Tesque Inc.), 1mM EDTA2Na, pH 8.0]. The suspension was frozen at -20 °C, and subsequently melted at 60 °C. 10mL of proteinase K solution (10 mg/mL) was added thereto. The mixture was allowed to stand at 37 °C for 10 minutes, and PCR reaction was carried out in the following manner using the mixture as DNA templates.

[Table 3]

**Table 3. Composition of PCR Reaction Fluid**

| Component | Quantity Contained (µL) |
|---|---|
| DNA Templates | 2.5 |
| 10×Ex taq Buffer | 5 |
| dNTP Mixture | 4 |
| Primer F(10pmol/µL)* | 2.5 |
| Primer R(10pmol/µL)* | 2.5 |
| Ex TaqDNA Polymerase | 0.25 |
| Water | 50 in total |

Note: Primer F: Note: Primer R: ATTACCGCGGCTGCTG (SEQ ID NO:2)

### <Temperature conditions>

(1) 95 °C, two minutes
(2) 35 cycles of the following
   95 °C, one minute
   55 °C, 30 seconds
   72 °C, 30 seconds
(3) 72 °C, five minutes

### <DGGE condition>

According to the manual of the Dcode Universal Mutation Detection System (mfd. by Bio-Rad), electrophoresis was conducted as follows.
Gel: 9% acrylamide gel
Denaturing gradient: 40-65%
Voltage: 60V
Time for electrophoresis: 17 hours

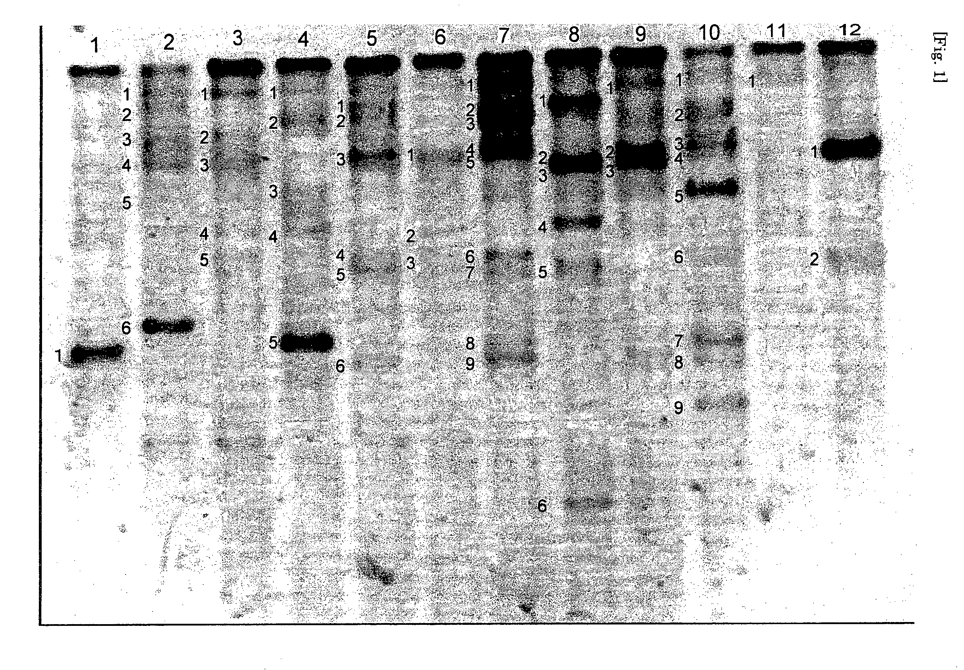

The result of electrophoresis is shown in Figure 1, and the search result using database search based on the sequence of DNA fragments obtained is shown in Table 4, respectively.

[Table 4]

**Table 4. Attribution of Bands (Lane No. - Band No.)**

| | |
|---|---|
| 1-1 Arthrobacter oxydans | 8-1 Cytophaga hutchinsonii |
| 2-1 Brevibacillus thermoruber | 8-2 Cohnella yongneupensis or Exiguobacterium indicum |
| 2-4 Sphingomonas sp. | |
| 2-5 Hirschia baltica | 8-5 Aquaspirillum sinuosum (Low homology) |
| 2-6 Spirosoma linguale | 8-6 Methylobacterium sp. |
| 3-4 Methylobacterium sp. | 9-1 Hirschia baltica |
| 4-3 Micrococcus luteus | 9-2 Methylocella palustris |
| 4-5 Micrococcus indicus | 9-3 Spirulina sp. (Low homology) |
| 5-3 Methylocella palustris | 10-1 Sphingopyxis sp. |
| 5-4 Micrococcus indicus | 10-2 Bacillus sp. |
| 5-5 Achromobacter sp. | 10-3 Methylocapsa acidiphila |
| 5-6 Xanthobacter aminooxidans (Low homology) | 10-4 Methylocella palustris |
| | 10-5 Acinetobacter sp., and others |
| 7-1 Spirosoma-like sp. | 10-6 Dyadobacter hamtensis |
| 7-5 Acinetobacter sp. | 10-7 Arthrobacter oxydans |
| 7-6 Achromobacter sp. | 10-9 Dyadobacter hamtensis |
| 7-7 Phaeospirillum molischianum | 11-1 Spirosoma linguale |
| 7-8 Sanguibacter sp. | 12-1 Spirulina sp. (Low homology) |
| 7-9 Streptomyces thermovulgaris | 12-2 Polaromonas sp. |

As a result, it was found that a number of methanol-utilizing bacteria such as Methylobacterium, Methylocella, Methylocapsa and the like, which were not observed by the above culture, were included. As for these methanol-utilizing bacteria, there is no report so far, to the knowledge of inventors, that they have been detected in Racomitrium canescens. The reasons for that seem that methanol-utilizing bacteria hardly grow in a culture medium rich in nutrients, and their growth therefore is slower than other bacteria. The fact that methanol-utilizing bacteria have been detected in Racomitrium canescens by the DGGE method suggests that methanol-utilizing bacteria are present preferentially with Racomitrium canescens.

According to the above result, in order to collect methanol-utilizing bacteria present with Racomitrium canescens, the above each sample of Racomitrium canescens was suspended in sterilized water, and inoculated to a culture medium which contained methanol as the carbon source (Table 5, referred to herein as "methanol culture medium"), and isolation of bacterial colony was attempted.

[Table 5]

**Table 5. Composition of Culture Medium for Methanol-utilizing Bacteria (in 1ml)**

| Component | Quantity Contained |
|---|---|
| (NH₄)₂HPO₃ | 0.3mg |
| Potassium Chloride | 0.1mg |
| Yeast Extract | 0.05mg |
| 1%MgSO₄/7H₂O | 10µL |
| Vitamin Solution (Table 4) | 10µL |
| Metal Solution (Table 5) | 10µL |
| Methanol | 1%(after autoclaved) |

[Table 6]

**Table 6. Composition of Vitamin Solution**

| Component | Quantity Contained (g/L) |
|---|---|
| Calcium Panthothenate | 0.4 |
| Inositol | 0.2 |
| Nicotinic Acid | 0.4 |
| p-aminobenzoic acid | 0.2 |
| Pyridoxine Hydrochloride | 0.4 |
| Thiamine Hydrochloride | 0.4 |
| Biotin | 0.2 |
| Vitamin B₁₂ | 0.2 |

[Table 7]

**Table 7. Composition of Metal Solution (in 1 L)**

| Component | Quantity Contained (g/L) |
|---|---|
| CoCl₂/6H₂O | 1.9 |
| MnCl₂/6H₂O | 1.0 |
| ZnCl₂ | 0.7 |
| H₃BO₃ | 0.06 |
| Na₂MoO₄/2H₂O | 0.36 |
| NiCl₂/6H₂O | 0.24 |
| CuCl₂/2H₂O | 0.02 |

As a result of the above culture, total seven strains were isolated as methanol-utilizing bacteria. 16S rDNA of each strain was analyzed, and the results were compared with those of strains which are open to the public on databases. Genus and species of each strain and sequence of each 16S rDNA are shown. (Provided that, only several hundred bases of the first half were determined for 21A and 41A.)
(1) MC-11A: Methylobacterium extorquens, 16S rDNA sequence: SEQ ID NO:3
(2) MC-11B: Methylobacterium extorquens, 16S rDNA sequence: SEQ ID NO:4
(3) 21A: It is closely related to bacteria of genus Spirosoma, 16S rDNA: SEQ ID NO:5
(4) MC-21B: Methylobacterium extorquens, 16S rDNA sequence: SEQ ID NO:6
(5) MC-21C: Methylobacterium extorquens, 16S rDNA sequence: SEQ ID NO:7
(6) MA-22A: Methylobacterium aquaticum, 16S rDNA sequence: SEQ ID NO:8
(7) 41A: It is closely related to bacteria of genus Mesorhizobium, 16S rDNA: SEQ ID NO:9

### [Study on effect of the isolated bacteria on growth of Racomitrium canescens]

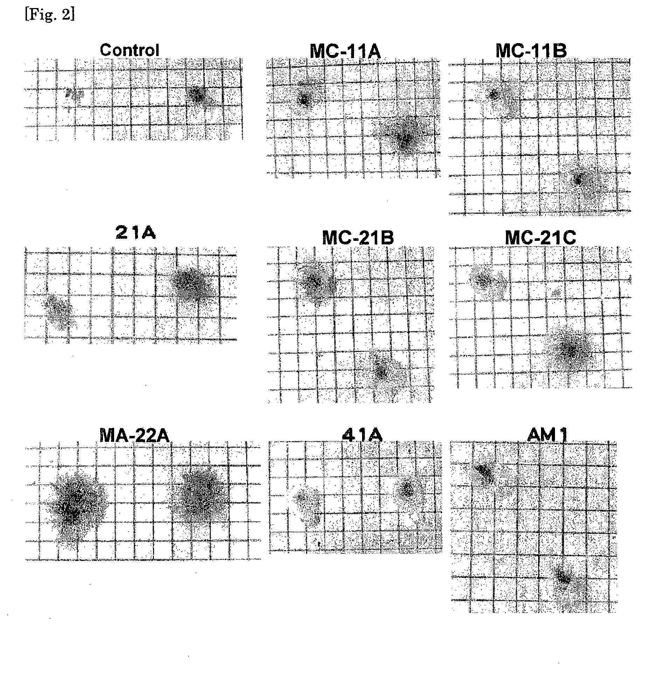

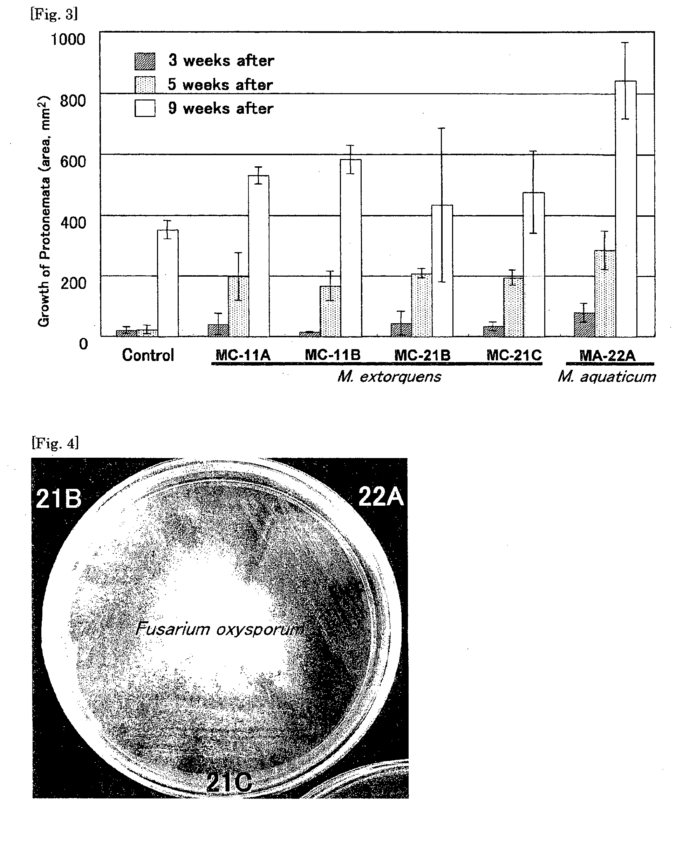

Protonemata which had been grown in Culture Medium Y (containing 0.8% agar) beforehand were picked up with tweezers one by one, and transferred onto two points of fresh Culture Medium Y (containing 0.8% agar). Colonies (approximately 1mm in diameter) of the above bacteria which had been grown on the methanol agar medium were collected with a sterilized platinum loop, and suspended in 300 µL of sterilized water, and 10 µL each of the suspension were inoculated to the planted protonemata, and then the medium was cultured under a fluorescent lamp at 20 °C. For purpose of comparison, the bacterium of Methylobacterium extorquens AM1 strain (ATCC14718) obtained from the culture collection was inoculated to protonemata in the same manner. Also, as a control, protonemata without inoculation of bacteria were cultured in the same manner. Results of 40 days of cultivation are shown in Figure 2. As seen in the figure, evident promotion of growth of the Racomitrium canescens was observed which had been inoculated with MA-22A, MC-21B or MC-21C when compared to the control, and the promotion of growth was particularly remarkable in moss inoculated with MA-22A. In contrast, growth promotion was not seen in protonemata inoculated with AM1 strain which was one of already known strains of Methylobacterium extorquens. Further, degree of growth was digitized in terms of elliptical area surrounding each protonemata at time points of 3 weeks, 5 weeks and 9 weeks after the start of the study, for each protnemata inoculated with MC-11A, MC-11B, MC-21B, MC-21C and MA-22A and protonemata without inoculation of bacteria (control) (Figure 3) (Error bars represent standard deviation. The same also applies in other graphs.) As a result, it was confirmed again that growth of protonemata inoculated with MA-22A is particularly outstanding, and it was also revealed that among other bacteria, MC-11A and MC-11B as well as MC-21B and MC-21C have growth-promoting effect.

### [Study on characteristics of isolated bacteria]

The separated bacteria were studied on properties which were considered to be important in interactions with plants. That is, nitrogen fixation ability, siderophore (siderophore: a ferric chelating substance) secretion, ability of indoleacetic acid synthesis from tryptophan (Trp), dissolution ability of insoluble calcium phosphate, beta-glucanase secretion ability, presence of the HCN (which has growth inhibitory effect on moulds) secretion ability, and presence of growth inhibitory effect on moulds were examined concerning the separated bacteria, as follows.

### <Testing method>

### (1) Nitrogen fixation ability:

Bacteria were streaked on an agar medium of the following composition which did not include nitrogen source, and growth was observed at 28 °C in the dark. Nitrogen fixation ability was determined to be positive if growth was observed.

[Table 8]

**Table 8. Composition of Agar Medium without Nitrogen Source (in 1 L. pH7.0)**

| Component | Quantity Contained |
|---|---|
| K₂HPO₄ | 1g |
| MgSO₄/7H₂O | 0.2g |
| CaCO₃ | 1g |
| NaCl | 0.2g |
| FeSO₄/7H₂O | 5mg |
| Agar | 15g |
| Glucose | 10g (sterilized separately, and added after autoclaved) |

### (2) Siderophore secretion:

According to Anal. Biochem, 160, 47-56, 1987, a test was conducted as follows. That is, at first, the following Solutions (A) to (C) were prepared.
(A) 60.5 mg of CAS (chrome azurol S) was dissolved in 50 mL of water, and mixed with 10 mL of 1 mM FeCl₃ solution in 10 mM hydrochloric acid. To the solution thus prepared was added slowly a solution of 72.9 mg of HDTMA (hexadecyltrimethylammonium bromide) dissolved in 40 mL of water.
(B) 750 mL of Water , 100 mL of 10 × MM9 salts (KH₂PO₄: 3 g/L, NH₄Cl: 10 g/L, NaCl: 5 g/L, MgSO₄: 2 mM, CaCl₂: 1 mM), 15 g of agar, and 30.24 g of PIPES [piperazine-1,4-bis(2-ethanesulfonic acid)] were mixed, and the pH was adjusted to 6.8 with sodium hydroxide aqueous solution.
(C) 30mL of 10% casamino acid aqueous solution.
After the above Solutions (A) to (C) were separately sterilized by autoclaving, cooled to about 50 °C, and mixed with each other. To this was added methanol at the final concentration of 1%, and the mixture was allowed to solidify in petri dishes. The plates thus obtained were blue in color. On the plates were streaked bacteria to be tested, and their growth was observed (at 28 °C, in the dark). In this culture medium, siderophore-positive bacteria will form colonies, the areas around which will lose their blue color and turn pink or transparent. Siderophore-negative bacteria will not grow in this culture medium.

### (3) Ability of indoleacetic acid synthesis from tryptophan:

According to Applied environmental microbiology, 1995, 61, 793-796, a test was conducted as follows. That is, bacteria to be tested were inoculated to King's B liquid culture medium (Difco proteose peptone No.3: 20 g/L, K₂HPO₄: 1.15 g/L, MgSO₄: 1.5 g/L, methanol: 1%, tryptophan: 2.5 mM). The culture medium was cultured with shaking at 28 °C in the dark. After growth was observed, the culture was centrifuged, and the supernatant was obtained as a sample. To one mL of the sample, one mL of Solution R1 (FeCl₃ in 7.9M H₂SO₄: 12 g/L) was added. The mixture was left to stand at room temperature for 30 minutes, and then, absorbance at 530nm was measured. A culture medium without inoculation of bacteria was used as a blank, and indoleacetic acid synthetic ability was determined as positive if color appears in comparison with the blank.

### (4) Dissolving ability of insoluble calcium phosphate:

According to FEMS Microbiol, Lett, 170, 265, 1999, a test was conducted as follows. That is, using NBRIP culture medium [National Botanical Research Institute's phosphate growth medium: glucose 10 g/L, Ca₃(PO₄)₂: 5 g/L, MgCl₂: 5 g/L, MgSO₄: 0.25 g/L, KCl: 0.2 g/L, (NH₄)₂SO₄: 0.1 g/L, pH 7, 1.5% agar], bacteria to be tested were streaked on the agar medium, and their growth was observed. The culture medium is cloudy (calcium phosphate), and growth and loss of the color of the medium will be observed if the bacteria have dissolving ability of calcium phosphate.

### (5) β-glucanase secretion ability:

Azurin-dyed and cross-linked beta-glucan was added at the concentration of 0.2% to commercially available R2A agar medium, and the mixture was allowed to solidify after autoclaving. The culture medium turned blue, but the substrate was not dissolved. Bacteria to be tested were streaked on the medium, and judged as positive if dispersion of dissolved substrate was observed around colonies.

### (6) HCN secretion ability:

According to MPMI vol 16, 2003, 525-535, a test was conducted as follows. That is, bacteria to be tested were inoculated to Castric's culture medium (Can J. Microbiol. 21, 613-618, 1975, L-glutamic acid: 40 mM, glycine: 10 mM, L-methionine: 10 mM, MgSO₄: 2 mM, NaH₂PO₄: 5 mM, K₂HPO₄: 5 mM, FeCl₃: 20 µM, pH 7.0), cultured with shaking at 28 °C in the dark. Test tubes which could be capped were used. After growth of bacteria was observed, the culture was centrifuged, and the supernatant was diluted 10 folds with water, and 50µL of this dilution was mixed with 250 µL of 0.1M NaOH. To this was added 0.5mL of 0.1M o-dinitrobenzene solution in 2-methoxyethanol, and, after 15 seconds, 0.5mL of 0.2M 4-nitrobenzaldehyde in 2-methoxyethanol was added, and then, 30 minutes later, absorbance at 578nm was measured. Simultaneously, 1 - 16 µM KCN solutions were treated in the same manner, and were made standard.

### <Results>

The results of the tests are shown in the following Table.

[Table 9]

**Table 9.**

| Strain | Attribution | Nitrogen Fixation Ability | Siderophore Secretion | Ability of Indoleacetic Acid Synthesis from Trp | Dissolution Ability of Insoluble Calcium Phosphate | ß-glucanase Secretion Ability | HCN Secretion Ability |
|---|---|---|---|---|---|---|---|
| MC-11A (FERM BP-11071) | *Methylobacterium extorquens* | + | + | + | + | - | + |
| MC-11B | *Methylobacterium extorquens* | + | + | + | + | - | + |
| MC-21B (FEFM BP-11079) | *Methylobacterium extorquens* | + | + | + | + | - | + |
| MC-21C (FERM BP-11080) | *Methylobacterium extorquens* | + | - | + | - | - | + |
| MA-22A (FERN BP-11078) | *Methylobacterium aquaticum* | + | - | + | - | - | ++ |
| 41A | *Mesorhizobium sp.* | + | - | - | + | - | - |
| 21A | Spirosoma sp. | + | - | - | - | + | - |
| AM1 (ATCC 14718) | *Methylobacterium extorquens* | + | + | + | + | - | not tested |

### (7) Growth inhibition effect on moulds:

White filamentous mould which grew on the surface of Racomitrium canescens were separated, and identified as Fusarium oxysporum by sequencing 18S rRNA genes according to a conventional method. This mould was planted on the middle of a R2A agar (Beckton-Dickinson and Company) plate. Isolated MA-22A, MC-21B and MC-21C were streaked around the mold planted, respectively, and the plate was cultured at 28 °C for about 5 days. It was found that bacterium MA-22A strongly inhibited the mold hyphae from spreading over the agar. The state is shown in Figure 4.

### [Example 2] Growth-promotion of Racomitrium canescens (2)

Ten g/L of sucrose was added to the following Knop+B5 vitamin culture medium, and after the pH of the mixture was adjusted to 5.8, 1.0 w/v % agar was added to prepare a culture medium for Racomitrium canescens.

[Table 10]

**Table 15. Knop + B5 Vitamin Culture Medium (in 100 mL)**

| Component | Quantity Contained (mg) |
|---|---|
| KH₂PO₄ | 25 |
| MgSO₄-7H₂O | 25 |
| KCl | 25 |
| Ca(NO₃)·4H₂O | 100 |
| FeSO₄·7H₂O | 1.25 |
| Thiamine Hydrochloride | 1.0 |
| Myo-Inositol | 10 |
| Pyridoxine Hydrochloride | 0.1 |
| Nicotinic Acid | 0.1 |

Protonemata of Racomitrium canescens were cultured for about 1.5 months at 23 °C under light for 24 hours a day in a 300-mL conical flask containing 100 mL of the culture medium for Racomitrium canescens described above, and this brought a state where the surface of the culture medium was covered with Racomitrium canescens. The Racomitrium canescens was collected from the surface of the culture medium, rinsed with tap water, and two flasks of it were put in a blender (mfd. by Waring) containing 45 mL of purified water, and was broken down into fragments at 10000 rpm for 60 seconds. One mL of this liquid containing fragmented Racomitrium canescens was applied on culture soil prepared by molding vermiculite with cellulose fibers (Florialite: San-ei Co., Ltd). The Florialite had been sterilized by autoclaving before use. After the liquid containing fragmented Racomitrium canescens was applied, the Florialite was saturated with a diluted commercially available liquid fertilizer (Brand name Hyponex). Methylotrophic bacteria, MA-22A, MC-11A and MC-21C, which had been cultured to cover R2A agar medium in the petri dishes were used as inoculation sources. The bacteria were collected with a spatula, suspended in 500 µL of sterilized water, and then 50 µL of the suspension were inoculated to each Florialite to which the liquid of fragmented Racomitrium canescens had been applied. Moss was prepared which had been inoculated with no bacteria and was called Control 1, and, also, moss was prepared to which liquid fertilizer containing 3 mg/L kinetin had been applied without inoculation with bacteria was called Control 2. After the transplanting, all the Racomitrium canescens was acclimated to the same condition in an acclimating room with lighting apparatus while being humidified in order not to be dried.

After two weeks from the start of acclimation, the moss which had been inoculated with methylotrophic bacterium still kept their green color that was brighter than either of Control 1 or 2. In Control 1, the survival rate of the moss was low, and the gametophore formation was very limited. On the other hand, in Control 2, which received kinetin treatment alone, the survival rate of the moss was higher than Control 1, and gametophore formation was also well induced. Also, in the moss which was inoculated with the methylotrophic bacterium, survival rate was higher than Control 1, and gametophore formation was well induced like Control 2. Particularly, the moss inoculated with MC-11A strain showed the best growth and gametophore differentiation. Thus, survival rate and gametophore formation rate after acclimation were raised by inoculating methylotrophic bacterium.

### INDUSTRIAL APPLICABILITY

Since this invention enables fast growth of protonemata of Racomitrium canescens, it will open a way to an efficient and increased production of Racomitrium canescens, enabling a wide variety of utilizations of Racomitrium canescens as greening material.

## Claims

1. A bacterium selected from methanol-utilizing bacteria of the genus Methylobacterium which bacteria are deposited under designated accession numbers FERM BP-11078, FERM BP-11079, FERM BP-11080 and FERM BP-11071, respectively.

2. A composition comprising bacterium of Claim 1 and protonemata of Racomitrium canescens.

3. A composition according to Claim 2, wherein the bacterium and the protonemata are contained in a culture medium.

4. A method for growth promotion of protonemata of Racomitrium canescens, which method comprises a step of providing at least one of methanol-utilizing bacteria of the genus Methylobacterium which bacteria are deposited under designated accession numbers FERM BP-11078, FERM BP-11079, FERM BP-11080, and FERM BP-11071, respectively, and a step of culturing protonemata of Racomitrium canescens together with the provided bacterium or bacteria.

## Patentansprüche

1. Bakterium, das aus Methanol-verwertenden Bakterien der Gattung *Methylobacterium* ausgewählt ist, wobei die Bakterien unter den zugewiesenen Zugangsnummern FERM BP-11078, FERM BP-11079, FERM BP-11080 bzw. FERM BP-11071 hinterlegt wurden.

2. Zusammensetzung, die das Bakterium nach Anspruch 1 und Protonemata von *Racomitrium canescens* enthält.

3. Zusammensetzung nach Anspruch 2, wobei das Bakterium und die Protonemata in einem Kulturmedium enthalten sind.

4. Verfahren zur Wachstumsförderung von Protonemata von *Racomitrium canescens,* wobei das Verfahren einen Schritt des Bereitstellens mindestens eines Bakteriums aus Methanol-verwertenden Bakterien der Gattung *Methylobacterium* umfasst, wobei die Bakterien unter den zugewiesenen Zugangsnummern FERM BP-11078, FERM BP-11079, FERM BP-11080 bzw. FERM BP-11071 hinterlegt wurden, sowie einen Schritt des Kultivierens von Protonemata von *Racomitrium canescens* zusammen mit dem bereitgestellten Bakterium oder den bereitgestellten Bakterien umfasst.

## Revendications

1. Bactérie choisie parmi les bactéries utilisant le méthanol du genre *Méthylobacterium* lesquelles bactéries sont déposées sous les numéros d'accès désignés FERM BP-11078, FERM BP-11079, FERM BP-11080 et FERM-BP-11071.

2. Composition comprenant la bactérie selon la revendication 1 et les protonemata de *Racomitrium canescens.*

3. Composition selon la revendication 2, dans laquelle la bactérie et les protonemata sont contenus dans un milieu de culture.

4. Procédé pour favoriser la croissance de protonemata de *Racomitrium canescens,* lequel procédé comprend une étape de fourniture d'au moins une parmi les bactéries utilisant le méthanol du genre *Méthylobacterium* lesquelles bactéries sont déposées sous les numéros d'accès désignés FERM BP-11078, FERM BP-11079, FERM BP-11080 et FERM-BP-11071, respectivement, et une étape de culture de protonemata de *Racomitrium canescens* avec la ou les bactéries fournies.
